# EUROPEAN PATENT SPECIFICATION

(11) **EP 2 773 750 B1**
(45) Date of publication and mention of the grant of the patent: **01.06.2016**
(21) Application number: 12784657.4
(22) Date of filing: 02.11.2012
(51) Int. Cl.: C12N 5/16, G01N 33/576

(54) **METHOD FOR REPLICATING HCV IN VITRO**
VERFAHREN ZUR REPLIKATION VON HCV IN VITRO
PROCÉDÉ POUR LA RÉPLICATION DE HCV IN VITRO

(30) Priority: 03.11.2011 GB 201119011
(43) Date of publication of application: 10.09.2014
(73) Proprietor: Queen Mary University of London, London E1 4NS (GB)
(72) Inventor: FOSTER, Graham, London E1 4AT (GB); CUNNINGHAM, M.E., London E1 4AT (GB); JAVAID, Alia, London E1 4AT (GB)
(74) Representative: D Young & Co LLP
(86) International application number: PCT/GB2012/052730
(87) International publication number: WO 2013/064833

(56) References cited:
- EP-A1- 0 414 475
- WO-A1-87/05930
- US-A1- 2006 154 235
- T ITO ET AL: "Acquisition of susceptibility to hepatitis C virus replication in HepG2 cells by fusion with primary human hepatocytes: Establishment of a quantitative assay for hepatitis C virus infectivity in a cell culture system", HEPATOLOGY, vol. 34, no. 3, 1 September 2001 (2001-09-01), pages 566-572, XP055050683, ISSN: 0270-9139, DOI: 10.1053/jhep.2001.26752
- CUNNINGHAM MORVEN E ET AL: "Development and validation of a novel capture-fusion model to study primary HCV replication and anti-viral drug sensitivity", HEPATOLOGY, vol. 56, no. Suppl. 1, October 2012 (2012-10), page 710A, XP002690857, & 63RD ANNUAL MEETING OF THE AMERICAN-ASSOCIATION-FOR-THE-STUDY-OF-LIVE R-DISEASES (AASLD); BOSTON, MA, USA; NOVEMBER 09 -13, 2012
- S. ALI ET AL: "Hepatitis C Virus Subgenomic Replicons in the Human Embryonic Kidney 293 Cell Line", JOURNAL OF VIROLOGY, vol. 78, no. 1, 1 January 2004 (2004-01-01), pages 491-501, XP055050677, ISSN: 0022-538X, DOI: 10.1128/JVI.78.1.491-501.2004
- RADKOWSKI MAREK ET AL: "Infection of primary human macrophages with hepatitis C virus in vitro: induction of tumour necrosis factor-alpha and interleukin 8.", THE JOURNAL OF GENERAL VIROLOGY, vol. 85, no. Pt 1, January 2004 (2004-01), pages 47-59, XP002690856, ISSN: 0022-1317

## Description

### FIELD OF THE INVENTION

The present invention relates to a method for replicating HCV virus *in vitro*, a cell capable of replicating HCV *virus in vitro* and its use to screen for drugs useful in HCV treatment.

### BACKGROUND TO THE INVENTION

Chronic infection with hepatitis C virus (HCV) infection is a global health problem affecting over 170 million people worldwide. At least 20% of those with chronic infection develop cirrhosis within 20-30 years and this progresses to a life threatening complication (liver failure or liver cancer) in >5% of individuals per year. In the UK, at least 250,000 people are infected, and the number with cirrhosis is predicted to more than double in the next decade, with a commensurate increase in the number with end stage liver disease or cancer.

Current therapies for chronic HCV infection involve a 24- to 48-week course of a long-lasting interferon (pegylated interferon [PEG-IFN]) combined with the oral antiviral agent, ribavirin. Therapy is unpleasant, with a range of side effects leading to early cessation of treatment in up to 20% of those receiving treatment. The outcome of therapy is heavily influenced by viral genotype (Gt). HCV persists as different genotypes with different geographical distributions - Gt3 being common in the Indian sub-continent and Gt4 in Egypt. In the US, Gt1 predominates but in Europe a mixed picture is seen - in the UK ~ 40% of patients are infected with Gt3. Up to 80% of patients with genotype (Gt) 2 respond to PegIFN and ribavirin therapy whilst <50% of those infected with Gt1 respond. In patients with Gt3, response rates are high (around 70%) but studies have shown that patients with cirrhosis have much lower rates of response. Patients with Gt4 have around a 60% chance of achieving an SVR with current therapies. For patients who respond to therapy (no virus in the circulation 24 weeks after therapy), liver damage resolves and most patients are considered cured, although the term 'sustained virological response' (SVR) is used to denote the uncertainty regarding long term prognosis in such patients.

### Screening_anti-HCV drugs in vitro

Given the relatively poor response to current therapies for chronic HCV and the massive and rising disease burden, there has been a global drive to develop new direct acting antiviral agents against HCV. Development has been hampered by the lack of robust *in vitro* screening tests for HCV. At present two laboratory models are widely used. One involves an unusual strain of HCV: the JFH-1 strain, a genotype 2 virus which propagates in hepatocyte derived cell lines (Huh7). The other laboratory models involve consensus, cloned HCV strains modified to form self replicating RNA species ('replicons'). These replicons adapt to tissue culture and can be manipulated to produce infectious virions. The replicons derived to date originate from Gt1a and 1b strains of HCV but their tissue culture adaptations render them non-infectious in chimpanzee models, leading many to question their reliability as a model of infectious virus. Hybrid viruses can be made that combine some elements of different genotypes but for regions of the tissue adapted viral strains that are unique (particularly the NS5B protein) fused viruses have not been generated. Thus, current models allow screening of drugs for activity against some viral genotypes (albeit using atypical viral strains) but it is not possible to test for activity against all genotypes and models for Gts 3 and 4 do not exist.

The shortcomings of current replication models are shown by the poor correlation between predicted activity and *in vivo* activity. The protease inhibitor telaprevir was predicted to inhibit genotype 3 HCV but, in patients, the drug has minimal activity. Likewise a compound in development by the pharmaceutical company Arrow inhibited the NS5A protein of HCV but had activity in patients only against certain genotype 1b substrains.

There is thus an acute need for a system that allows HCV of different genotypes to be grown in the laboratory.

### Current HCV Therapies

Direct acting antiviral agents (protease inhibitors) against HCV have been developed, such as telaprevir and boceprevir. The drugs are used in combination with PegIFN and ribavirin and have been reported to increase the proportion of patients with Gt1 HCV who respond to therapy. However the drugs have significant side effects - noticeably anaemia and rashes, which may progress to life-threatening Stevens-Johnson syndrome. The drugs are also expensive, costing around £25,000 per course. Given the costs and side effects of these new agents, attempts have been made to identify factors that predict the ~50% of patients with genotype 1 HCV who will respond to PegIFN and ribavirin. Two approaches have been used. One approach involves the use of a genetic polymorphism in the IL-28B non-coding region of the human genome: some haplotypes have a 70% response to PegIFN and ribavirin whilst others have a 30% response. An alternative approach involves administering PegIFN and ribavirin and measuring the response: patients whose virus is undetectable after 4 weeks ('rapid virological responders') may be cured by PegIFN and ribavirin alone. One attractive strategy to reduce needless use of protease inhibitors is to combine these markers (often with other patient characteristics, such as presence or absence of cirrhosis) and to withhold protease inhibitors from those predicted respond. Such an approach is likely to prove popular provided that the relapse rate after therapy is low - if relapse occurs patients are required to re-initiate therapy with a protease inhibitor negating the cost and side effect benefits of withholding the drugs. Published work indicates that the predictive value of IL-28 genotyping is not yet adequate for patient stratification but the response to pegylated interferon and ribavirin may be predictive with between 80 and 95% of patients who achieve a rapid virological response.

There is therefore a need for an improved method to predict an HCV patient's susceptibility to treatment with drugs such as PegIFN and ribavirin, telaprevir and boceprevir.

### Future therapies and drug resistance

Future therapy for HCV is likely to involve a combination of direct acting anti-viral agents and over 100 are currently in development. Since HCV replicates with a low fidelity polymerase enzyme, viral variants are ubiquitous and resistance to single agent drug therapy develops rapidly. Since these drug resistant variants are, to-date, sensitive to PegIFN it is probable that drug resistant HCV will not be unduly problematic provided PegIFN remains the backbone of therapy. However, as drug combinations that do not involve PegIFN and ribavirin develop it is likely that viral resistance will emerge as a significant problem and, particularly in patients previously exposed unsuccessfully to one or more direct acting antiviral agents, it is likely that pre-treatment prediction of response would be increasingly valuable. Pre-treatment viral phenotype testing to predict response to antiviral agents was used highly successfully in the development of effective regimes for HIV.

There is thus a need for an HCV replication model suitable for use in pre-treatment HCV viral phenotyping.

### HCV Relapse

Predicting the response to PegIFN and ribavirin is often confounded by virological relapse, when a patient who has undetectable HCV RNA in serum during treatment develops recurrent viraemia when therapy is discontinued. The fear of relapse persuades clinicians to continue therapy for many months after virus has become undetectable in serum and understanding the mechanisms of relapse may allow better modifications to treatment duration. For telaprevir, where relapse and viral breakthrough occurs in 6% more patients treated for 8 rather than 12 weeks, understanding and monitoring factors underlying relapse would allow early treatment discontinuation in patients deemed likely not to relapse.

There is therefore a need for an improved method to predict the likelihood of an HCV patient to relapse at a given point in treatment, if the treatment were to be stopped.

### DESCRIPTION OF THE FIGURES

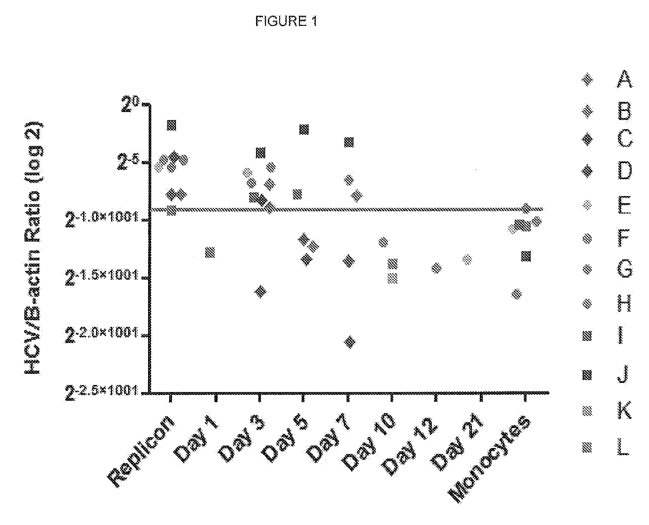

**Figure 1** **-** Summary of fusions with Huh 7.5s patient derived monocytes
   Patient derived monocytes from 12 patients with chronic HCV (labelled A - L) were fused to a hepatocyte cell line and after the indicated days the amount of HCV RNA was detected using quantitative PCR. The quantity of HCV is expressed relative to Beta Actin. Controls are monocytes alone from the indicated patients and a genotype 1b replicon.
**Figure 2** **-** HCV Replication
   Patient derived moncytes from 13 patients with chronic HCV were fused to an hepatocyte cell line and after the indicated days the amount of HCV RNA was detected. The quantity of HCV is expressed relative to Beta Actin mRNA. Results are expressed after less than 7 or more than 7 days incubation.
**Figure 3** **-** HCV Replication compared to monocytes
   Patient derived monocytes from 4 patients with chronic HCV were fused to a hepatocyte cell line and after 3 days the amount of HCV RNA was detected using quantitative PCR. White blood cells from the same patients were tested prior to fusion and the paired samples are linked graphically.
**Figure 4** **-** Fusions: genotypes 1, 2 and 3
   Patient derived monocytes from 8 patients with chronic HCV were fused to a hepatocyte cell line and after the indicated days the amount of HCV RNA was detected using quantitative PCR. Four of the patients were infected with HCV genotype 1, one patient was infected with HCV genotype, and three patients were infected with HCV genotype 3. * *P* = <0.05 compared to unfused monocytes (*P* = 0.037 for day 3 comparison; *P* = 0.038 for day 5 comparison)

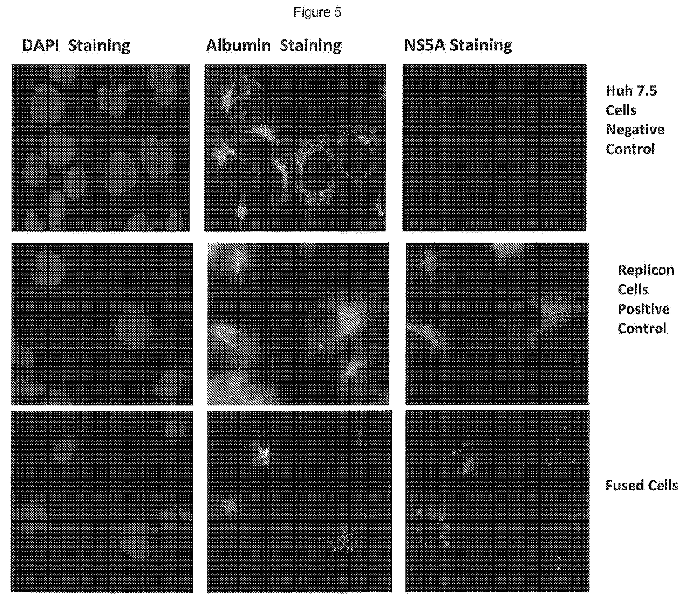

**Figure 5** **-** Immunofluorescence microscopy
   Immunofluorescence microscopy of fused monocytes from a patient without or with chronic HCV infection. Five days after fusion the fused cells were stained with a commercial monoclonal antibody against human albumin and an antibody directed against the NS5A protein of HCV.

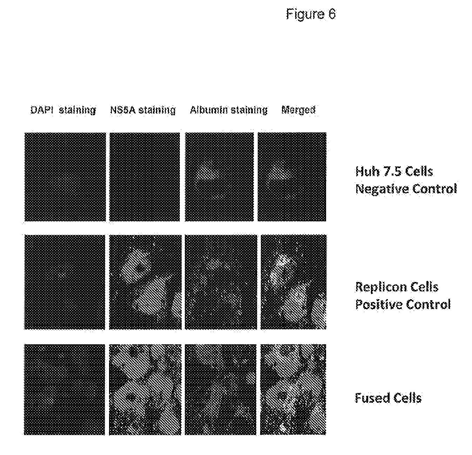

**Figure 6** - Confocal microscopy
   Confocal microscopy of fused monocytes from a patient without or with chronic HCV infection. Five days after fusion the fused cells were stained with a commercial monoclonal antibody against human albumin and serum directed against the NS5A protein of HCV. Images were fused to generate fused confocal images.

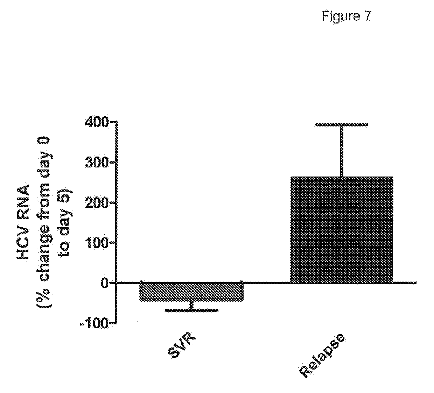

**Figure 7** **-** Presence of viable HCV RNA in mnoncytes at end of treatment predicts relapse in patients with G3 HCV
   Monocytes from 20 patients with genotype 3 HCV taken at the end of treatment were isolated and fused to human hepatocytes. After a few hours and 5 days mRNA was prepared and HCV RNA was detected. Results are expressed as the percentage change at 5 days compared to day 0 (i.e. day of fusion).

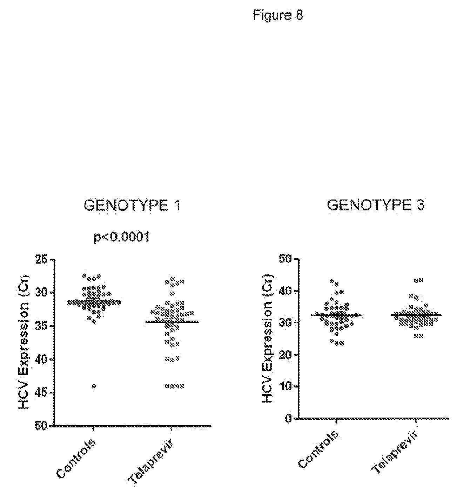

**Figure 8** **-** Effect of telaprevir on HCV Genotype 1 and genotype 3

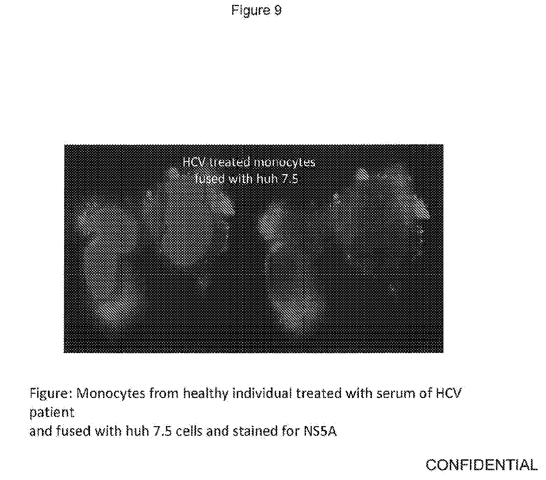

**Figure 9** **-** Transfer of HCV via monocytes Monocytes from a patient with HCV were fused to hepatocytes and stained for HCV using an antibody directed against NS5A

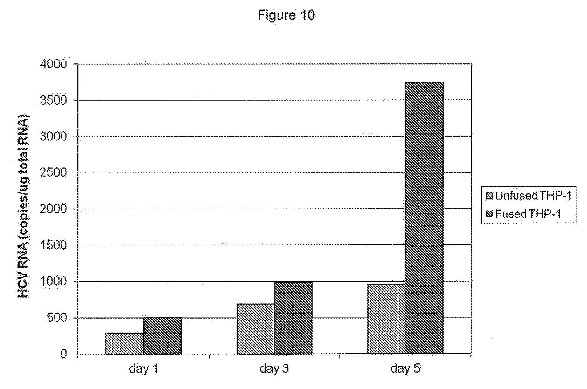

**Figure 10** **-** Fusion of infected THP-1 cells enhances HCV replication
   ThP1 cells in culture were cultured with serum from a patient with HCV and then either left unfused or fused to a hepatocyte cell line. HCV RNA was then assessed in the total cellular mRNA. HCV RNA was quantified by using a cloned HCV cDNA diluted to a known concentration and used to generate a standard curve. The quantity of HCV RNA in the samples was calculated by comparing the Ct values to the Ct values from the standard curve.

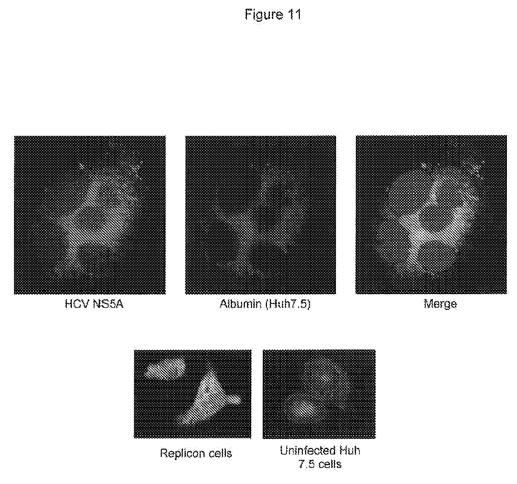

**Figure 11** **-** Transfer of HCV via monocytes
   Confocal microscopy of fused ThP1 cells after incubation with serum from a patient with HCV followed by fusion with an hepatocyte cell line. The fused cells were stained with a commercial monoclonal antibody against human albumin and an antibody directed against the NS5A protein of HCV. Images were fused to generate fused confocal images.

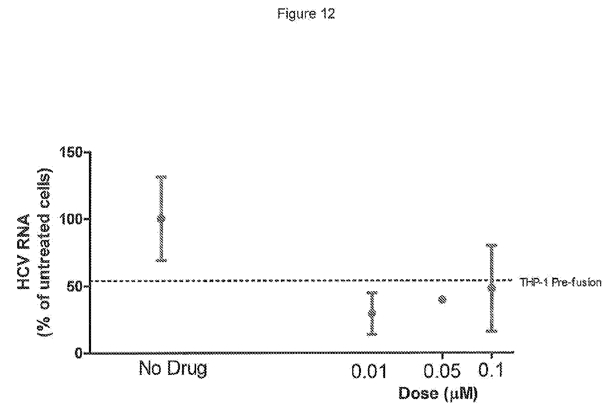

**Figure 12** **-** Effect of Anti-viral compound A on fused cells made from Genotype 3 infected THP-1 cells

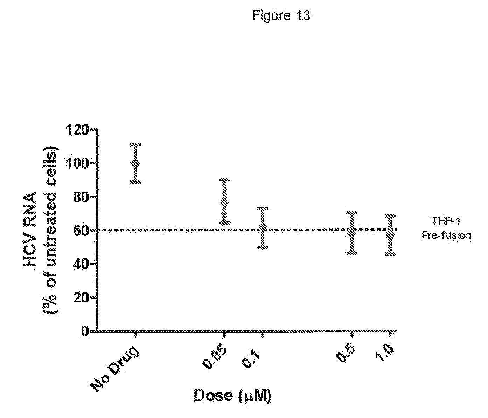

**Figure 13** **-** Effect of Anti-viral compound A on fused cells made from Genotype 1 infected THP-1 cells

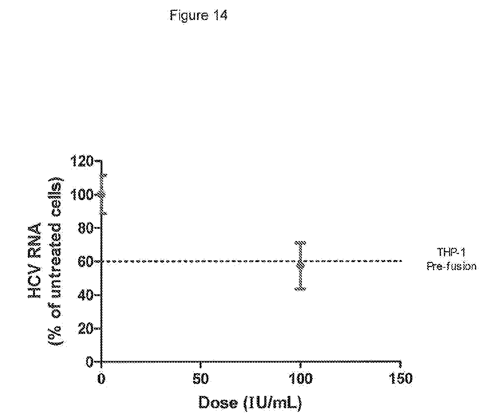

**Figure 14** **-** Effect of IFN on fused cells made from Genotype 1 infected THP-1 cells
   The fused cells were treated for 5 days with the indicated concentrations of IFN alpha 2a.

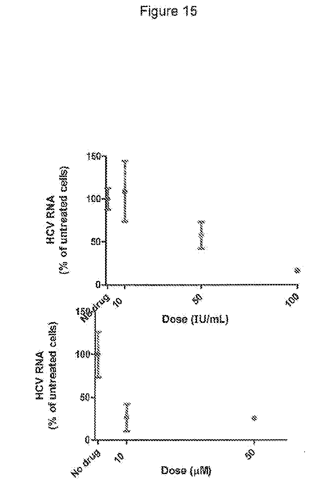

**Figure 15** **-** Effect of IFN on fused cells made from Genotype 3 infected THP-1 cells

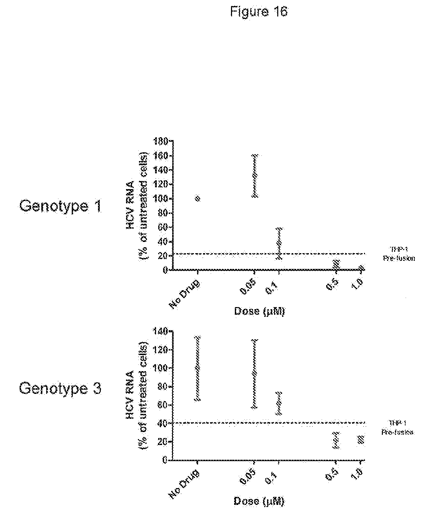

**Figure 16** **-** Effect of Telaprevir on fused cells made from Genotype 1 and 3 infected THP-1 cells

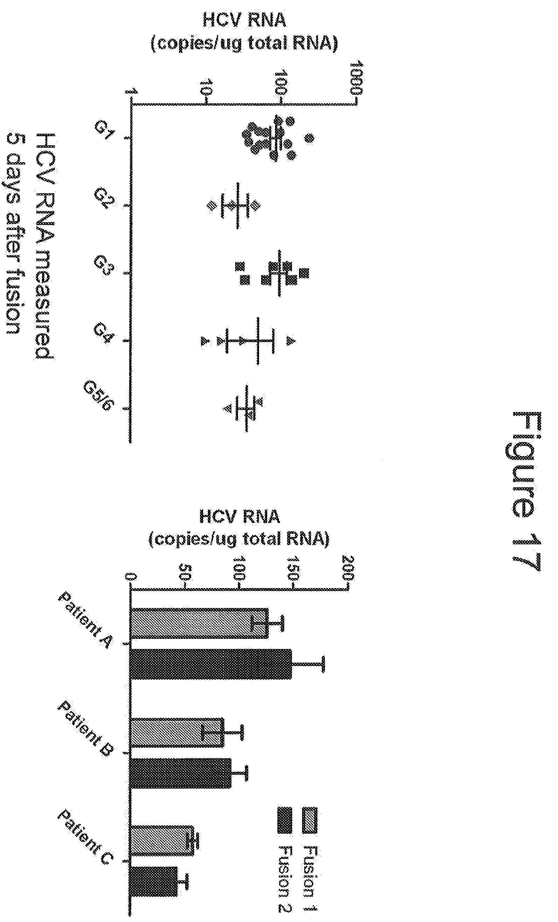

**Figure 17** **-** HCV RNA can be quantified from all naive patients

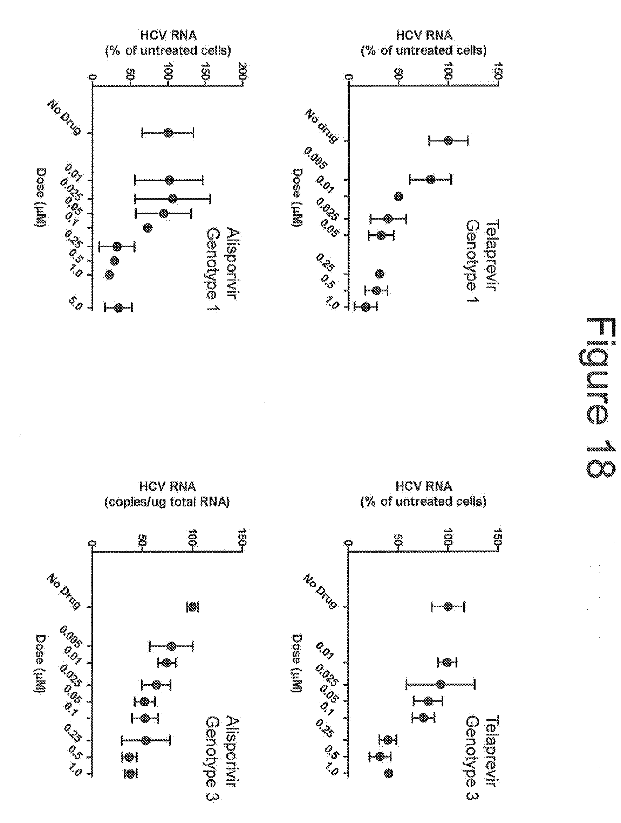

**Figure 18** **-** Individual patient dose-response curves of different genotypes following treatment with Telaprevir or Alisporivir

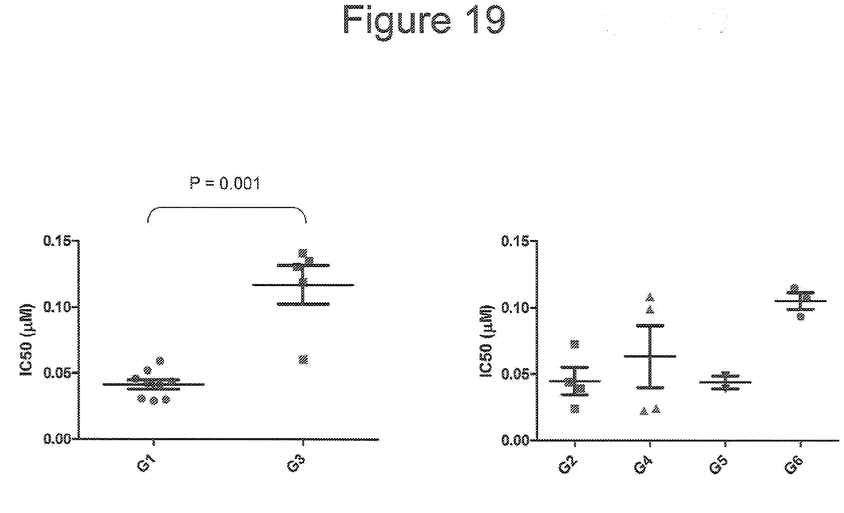

**Figure 19** **-** Investigation of Telaprevir sensitivity in a variety of different viral genotypes

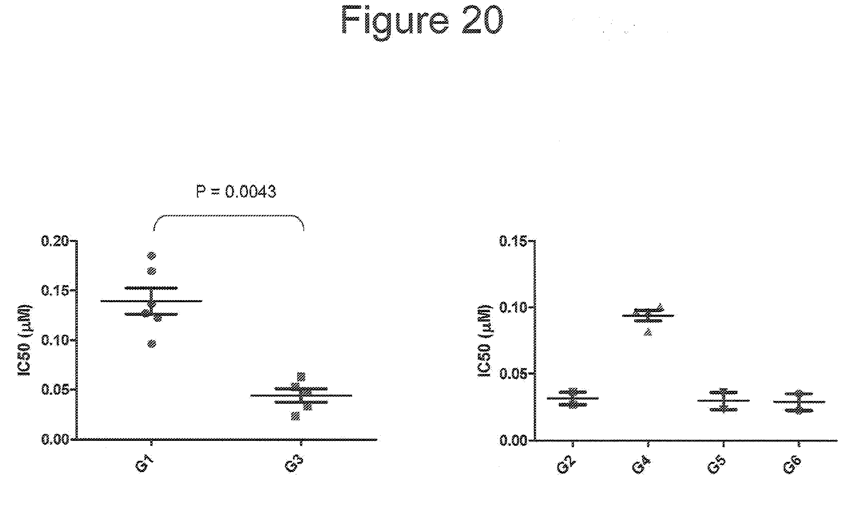

**Figure 20** **-** Investigation of alisporivir sensitivity in a variety of different viral genotypes

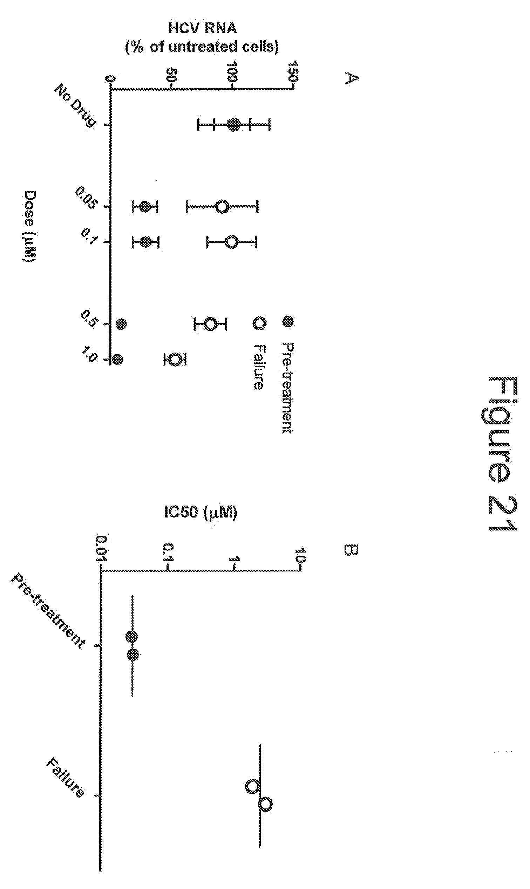

**Figure 21** **-** Reduced telaprevir sensitivity after acquisition of telaprevir resistance associated NS3 mutations. Graph A shows mean±sem of capture-fusion experiments using pre-treatment and post-failure samples from 2 patients who failed therapy with telaprevir PegIFN and ribavirin. Both had wild-type NS3 pre-treatment and dual V36M/R155K telaprevir resistance mutations at treatment failure. Graph B shows telaprevir IC₅₀ for each patient before telaprevir treatment and after treatment failure.

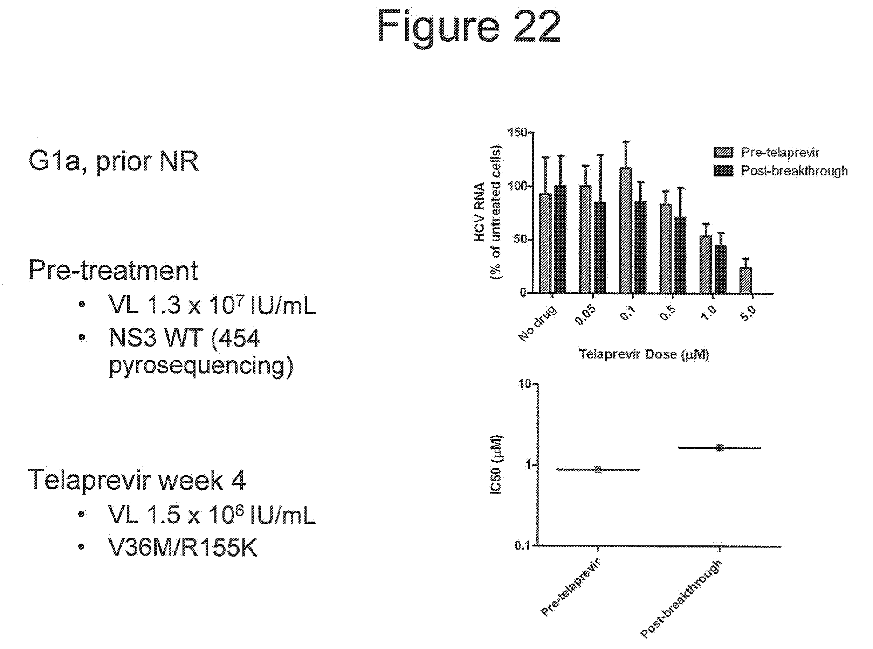

**Figure 22** **-** Identification of pre-existing telaprevir resistance despite wild-type NS3

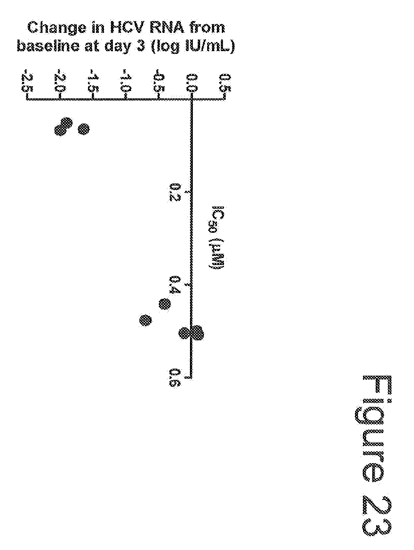

**Figure 23** **-** Identification of pre-treatment telaprevir sensitivity in patients with G3 HCV Correlation between pre-treatment telaprevir sensitivity using the capture-fusion assay and clinical response to telaprevir monotherapy in patients with G3 HCV. X axes show sensitivity to telaprevir (IC50) in each assay and y axes show clinical response to telaprevir monotherapy. Graph A, Spearman correlation coefficient 0.88, p = 0.007.

### SUMMARY OF ASPECTS OF THE INVENTION

The present inventors have found that fusing monocytes from patients with chronic HCV to a hepatocyte cell line (HuH7) leads to replication of HCV in the fused cells for over a week. They have adapted this approach into a 'capture-fusion' approach where infected sera from HCV patients are incubated with monocyte cell lines that are then fused to hepatocytes. This approach makes it possible to investigate *in vitro* the drug sensitivity of circulating viruses from patients.

Thus in a first aspect the present invention provides a method for replicating HCV virus *in vitro* which comprises the following steps:
(i) fusing an HCV-infected white blood cell with a hepatocyte cell to produce a fused cell; and
(ii) culturing the fused cell so that HCV replication may occur.

In a first embodiment of this aspect of the invention, the HCV-infected white blood cell is isolated from an HCV patient.

In a second embodiment of this aspect of the invention, the HCV-infected white blood cell is made by infecting a white blood cell with HCV virus *in vitro.* In this embodiment that white blood cell may be derivable from a white blood cell (e.g. a monocyte) cell line. The white blood cell may be pre-treated with at least one pro-inflammatory reagent prior to infection *in vitro.* The white blood cell may be pre-treated with a cocktail of pro-inflammatory reagents. The pro-inflammatory reagent may be, for example, interferon γ (IFNγ) and/or PMA Alternatively the pro-inflammatory reagent may be lipopolysaccharide and other Toll Like receptor agonists including flagellin, imiquimod and IL-1.

The white blood cell may be any white blood cell which has the capacity to capture HCV virus. Suitable white blood cell types include: monocytes, polymorphs, macrophages and B cells.

The white blood cell may be infected with HCV virus having any HCV genotype. For example, the white blood cell may be infected with a non-1 genotype, such as genotype 2, 3, 4, 5 or 6 HCV.

In a second aspect the present invention provides a fused cell capable of replicating HCV virus *in vitro* which is made by fusing an HCV-infected white blood cell with a hepatocyte cell.

In a third aspect, the present invention provides a method for making a fused cell according to the second aspect of the invention, which method comprises the step of fusing an HCV-infected white blood cell with a hepatocyte cell *in vitro.*

In a first embodiment of the third aspect of the invention, the HCV-infected white blood cell is derived from a HCV patient.

In a second embodiment of the third aspect of the invention, the HCV-infected white blood cell is made by infecting a white blood cell with HCV virus *in vitro.*

The method may comprise the following steps:
(i) culturing a white blood cell in vitro with serum from an HCV patient, so that the white blood cell is infected with HCV in the serum; and
(ii) fusing the HCV-infected white blood cell with a hepatocyte.

In a fourth aspect, the present invention provides the use of a fused cell according to the second aspect of the invention to assess the capacity of a test HCV treatment to reduce HCV replication.

In a fifth aspect, the present invention provides a method for screening a test compound or composition which comprises the step of analysing the capacity of the test compound or composition to reduce HCV replication in a fused cell according to the second aspect of the invention.

The test compound or composition may, for example, comprise IFN and/or ribovarin.

The capacity of the test compound or composition may be tested in a plurality of fused cells according to the second aspect of the invention, each fused cell replicating a different strain of HCV virus, in order to analyse whether the effectiveness of the test compound or composition is HCV strain-specific.

In a sixth aspect, the present invention provides an *in vitro* method for assessing the likelihood that a HCV patient will respond to a test compound or composition, which comprises the step of analysing the capacity of the test compound or composition to reduce HCV replication *in vitro* in a fused cell,
wherein the fused cell is made by fusing a hepatocyte cell with
a white blood cell which is either:
(i) isolated from the HCV patient; or
(ii) a white blood cell which has been infected with HCV virus from the subject *in vitro*.

In a seventh aspect, the present invention provides an *in vitro* method for selecting a treatment which comprises the step of assessing the responsiveness of an HCV patient to a test compound or composition by a method according to the fifth aspect of the invention and selecting a test compound or composition which reduces HCV replication in the fused cell.

In an eighth aspect, the present invention provides an *in vitro* method for assessing the likelihood that a subject will relapse following treatment, which comprises the following steps:
(i) fusing a white blood cell which has been isolated from the subject with a hepatocyte cell to produce a fused cell; and
(ii) investigating whether HCV can be detected in the fused cell,
wherein a subject is determined to be likely to relapse if HCV is detectable in the fused cell.

The white blood cell may be a monocyte.

The presence of HCV may be investigated by detecting the presence or absence of HCV RNA.

In a ninth aspect, the present invention provides an *in vitro* method for investigating the progression of an HCV treatment which comprises the following steps:
(i) fusing a white blood cell such as a monocyte, which has been periodically isolated from the subject with a hepatocyte cell to produce a fused cell; and
(ii) investigating whether HCV can be detected in the fused cell.

In a tenth aspect, the present invention provides an *in vitro* method for determining when an HCV treatment may be stopped by investigating the progression of the treatment by a method according to the ninth aspect of the invention and determining that the treatment may be stopped when a white blood cell, such as a monocyte, is isolated which produces a fused cell in which HCV is undetectable.

### DETAILED DESCRIPTION

### HEPATITIS

Hepatitis is a medical condition defined by the inflammation of the liver and characterized by the presence of inflammatory cells in liver tissue.

Initial features are of nonspecific flu-like symptoms, common to almost all acute viral infections, and may include malaise, muscle and joint aches, fever, nausea or vomiting, diarrhea, and headache. More specific symptoms, which can be present in acute hepatitis from any cause, are: profound loss of appetite, aversion to smoking among smokers, dark urine, yellowing of the eyes and skin (i.e., jaundice) and abdominal discomfort.

Viral hepatitis may be acute or chronic.

Acute viral hepatitis is more likely to be asymptomatic in younger people. Symptomatic individuals may present after convalescent stage of 7 to 10 days, with the total illness lasting 2 to 6 weeks.

A small proportion of people with acute hepatitis progress to acute liver failure, in which the liver is unable to clear harmful substances from the circulation (leading to confusion and coma due to hepatic encephalopathy) and produce blood proteins (leading to peripheral edema and bleeding). This may become life-threatening and occasionally requires a liver transplant.

Chronic hepatitis often leads to nonspecific symptoms such as malaise, tiredness and weakness, and often leads to no symptoms at all. It is commonly identified on blood tests performed either for screening or to evaluate nonspecific symptoms. The occurrence of jaundice indicates advanced liver damage. On physical examination there may be enlargement of the liver.

Extensive damage and scarring of liver (i.e. cirrhosis) leads to weight loss, easy bruising and bleeding tendencies, peripheral edema (swelling of the legs) and accumulation of ascites (fluid in the abdominal cavity). Eventually, cirrhosis may lead to various complications: esophageal varices (enlarged veins in the wall of the esophagus that can cause life-threatening bleeding) hepatic encephalopathy (confusion and coma) and hepatorenal syndrome (kidney dysfunction).

### HEPATITIS C VIRUS (HCV)

### Hepatitis C virus (HCV) is the cause of hepatitis C in humans.

HCV is a small (55-65 nm in size), enveloped, positive-sense single-stranded RNA virus of the family *Flaviviridae.* The hepatitis C virus particle consists of a core of genetic material (RNA), surrounded by an icosahedral protective shell of protein, and further encased in a lipid envelope of cellular origin. Two viral envelope glycoproteins, E1 and E2, are embedded in the lipid envelope.

Based on genetic differences between HCV isolates, the hepatitis C virus species is classified into six genotypes (1-6) with several subtypes within each genotype (represented by letters). Subtypes are further broken down into quasispecies based on their genetic diversity. The preponderance and distribution of HCV genotypes varies globally. For example, in North America, genotype 1a predominates followed by 1b, 2a, 2b, and 3a. In Europe, genotype Ibis predominant followed by 2a, 2b, 2c, and 3a. Genotypes 4 and 5 are found largely in Africa, although there is a large Gt4 community in Spain. Genotype is clinically important in determining potential response to interferon-based therapy and the required duration of such therapy. Genotypes 1 and 4 are less responsive to interferon-based treatment than are the other genotypes (2, 3, 5 and 6). Duration of standard interferon-based therapy for genotypes 1 and 4 is 48 weeks, whereas treatment for genotypes 2 and 3 is completed in 24 weeks.

Infection with one genotype does not confer immunity against others, and concurrent infection with two strains is possible.

### HCV REPLICATION IN VITRO

In a first aspect, the present invention provides a method for replicating HCV virus *in vitro.* An HCV-infected white blood cell is fused with a hepatocyte cell to produce a fused cell which supports viral replication for over 7 days *in vitro.* The technique works with HCV viruses of all different genotypes.

Thus the HCV virus replicated using the method of the present invention may have any of the following genotypes: 1a, 1b, 2a, 2b, 2c, 3a, 4, 5 or 6 or any other genotye or sub-genotype The HCV virus may have a genotype other than genotype 1.

The HCV virus may have genotype 2, 3, 4, 5 or 6.

The method comprises the step of fusing an HCV-infected white blood cell with a hepatocyte cell to produce a fused cell.

The cells may be fuse by techniques known in the art such as PEG fusion, as described in the Examples.

### WHITE BLOOD CELL

The white blood cell may be any white blood cell which has the capacity to capture HCV virus. Suitable white blood cell types include: monocytes, polymorphs, macrophages and B cells.

Monocytes are a type of white blood cell which play multiple roles in the immune system. Their immunological roles include replenishing resident macrophages and dendritic cells, and migrating to sites of infection in response to inflammation signals where they divide/differentiate into macrophages and dendritic cells to elicit an immune response.

Monocytes are usually identified in stained smears by their large kidney shaped or notched nucleus.

Monocytes are produced by the bone marrow from haematopoietic stem cell precursors called monoblasts. Monocytes circulate in the bloodstream for about one to three days and then typically move into tissues throughout the body. They constitute between three to eight percent of the leukocytes in the blood.

Monocytes and their macrophage and dendritic-cell progeny serve three main functions in the immune system, namely phagocytosis, antigen presentation and cytokine production.

White blood cells may be isolated from patient samples, such as blood, tonsil, spleen or other tissue samples by techniques known in the art. For example, they may be isolated using bead selection as described in the Examples.

The white blood cell used to create the fused cell of the present invention is infected with HCV. This may happen *in vivo,* where a HCV-infected white blood cell is derived from an HCV patient. Alternatively, this may happen by infecting a white blood cell with HCV *in vitro.*

The present invention thus provides a 'capture-fusion' technique in which virus from patient's sera is captured by a white blood cell, which is then fused to a hepatocyte to generate a fused cell that supports viral replication *in vitro* for over 7 days. The technique works with HCV viruses of all different genotypes.

The white blood cell used in the "capture-fusion" technique may be derivable from a cell line, such as ThP1.

The white blood cell may be treated with at least one pro-inflammatory reagent prior to HCV infection *in vitro.* The white blood cell may be treated with a cocktail of pro-inflammatory reagent prior to HCV infection *in vitro.* For example, the white blood cell may be treated with PMA and/or IFNγ.

### HEPATOCYTE

In the method of the present invention, an HCV-infected white blood cell is fused with a hepatocyte.

The hepatocyte may be from a human hepatoma cell line.

The hepatocyte may be a HuH7 cell. HuH-7 is a well differentiated hepatocyte-derived cellular carcinoma cell line which is available from the JCRB cell bank (http://cellbank.nibio.go.jp/celldata/jcrb0403.htm).

### FUSED CELLS

The second aspect of the invention relates to a fused cell capable of replicating HCV virus *in vitro.*

The fused cell is made by fusing an HCV-infected white blood cell with a hepatocyte cell.

The fused cell may be capable of replicating HCV virus *in vitro* for about a week following cell fusion. The fused cell may be capable of replicating HCV *virus in vitro* for up to 5, 6, 7, 8 or 9 days following cell fusion.

Replication of HCV involves several steps. Once the virus has gained access to the cell, HCV takes over portions of the intracellular machinery to replicate. The HCV genome is translated to produce a single protein of around 3011 amino acids. The polyprotein is then proteolytically processed by viral and cellular proteases to produce three structural (virion-associated) and seven nonstructural (NS) proteins. The NS proteins then recruit the viral genome into an RNA replication complex, which is associated with rearranged cytoplasmic membranes. RNA replication takes places via the viral RNA-dependent RNA polymerase NS5B, which produces a negative-strand RNA intermediate. The negative strand RNA then serves as a template for the production of new positive-strand viral genomes. Nascent genomes can then be translated, further replicated, or packaged within new virus particles. New virus particles are thought to bud into the secretory pathway and are released at the cell surface.

HCV replication by the fused cell may be assessed by, for example, investigating HCV virion production by the fused cell or the presence of HCV RNA in the total cellular mRNA of the fused cell.

The fused cell may be capable of replicating HCV virus of any genotype.

Thus the HCV virus replicated by the fused cell of the present invention may have any of the following genotypes: 1a, 1b, 2a, 2b, 2c, 3a, 4, 5 or 6 or other, currently undesignated genotypes.

The HCV virus may have a genotype other than genotype 1.

The HCV virus may have genotype 2, 3,4, 5 or 6.

### SCREENING TEST TREATMENTS

The fused cell of the present invention may be used to screen test treatments for hepatitis C.

For example, a test treatment may be applied to a fused cell *in vitro* and the effect of the treatment on HCV replication examined. If the treatment reduces or blocks HCV replication in the fused cell *in vitro*, then it may be useful as a treatment of HCV infection *in vivo,* particularly infection with the same genotype or subtype as the HCV virus replicated by the fused cell.

The treatment may involve a single compound or a plurality of compounds. Where there is a plurality of compounds, they may be administered together, for example, in the form of a pharmaceutical composition; sequentially; or separately. The "treatment" may involve a treatment regime in which the dose and timing of administration of a particular compound or composition or a plurality of compounds or compositions is defined.

The treatment may involve administration of a known anti-HCV drug, such as IFN (or PEG-IFN), ribovarin, Telaprevir, alisporivir and/or boceprevir.

Alternatively, or in addition, the treatment may involve a potential new anti-HCV drug under development.

As mentioned in the Introduction section, model replication systems for genotype 1 and for a unique genotype 2 strain have been developed, but there are currently no *in vitro* models for the other viral strains. This means that the effectiveness of drugs against HCV with a genotype other than 1 can not be investigated by standard screening models it is necessary to test them directly in patients.

The method of the present invention may be used to screen new or existing drugs for antiviral activity against genotype non-1 HCV *in vitro.*

A large number compounds are have been developed commercially which show some activity against G1 HCV. The fused cell system of the present invention will allow the effectiveness of these 'back up' compounds to against other HCV strains to be investigated.

### INVESTIGATING HCV STRAIN SPECIFICITY

The fused cells of the present invention may be used to investigate the HCV strain specificity of a given Hepatitis C treatment. As mentioned above, it is known from standard IFN-based therapy that HCV-treatment may be strain selective. Genotype may affect both the response to interferon-based therapy and the required duration of the treatment. In this respect, HCV genotypes 1 and 4 are less responsive to interferon-based treatment than are the other genotypes (2, 3, 5 and 6).

Since the method of the invention may be used to produce a fused cell capable of replicating HCV virus of any genotype or subtype *in vitro,* a plurality of fused cells may be produced which replicate HCV virus of different genotypes or subtypes.

A treatment may then be tested on the plurality of fused cells in parallel and any differences in the effectiveness or duration of treatment examined. Assuming all else is equal, differences in the effectiveness or duration of treatment is thus attributable to differences in the HCV genotype or subtype.

### PATIENT STRATIFICATION

The HCV-infected white blood cell used to make a fused cell of the present invention may, for example, be a) directly derived from an HCV patient or b) made by infecting a white blood cell cell *in vitro* with HCV virus from a patient.

Thus the fused cell is effectively an *"in vitro* model" of an HCV-infected patient cell, which produces patient-derived HCV. This is extremely useful for screening for sensitivity to antiviral drugs prior to therapy.

The present invention therefore provides both an *in vitro* method for assessing the likelihood that a HCV patient will respond to a test treatment, and an *in vitro* method for selecting a treatment for use *in vivo* by selecting a treatment which reduces HCV replication in the fused cell model.

Another advantage of pre-treatment testing of drug is it reduces the number of times a patient is unsuccessfully treated with a drug. Unsuccessful drug treatment can cause the emergence of drug resistance as a virus adapts to avoid the administered drug.

### RELAPSE LIKELIHOOD

As mentioned above, current treatments for chronic HCV infection are costly and associated with deleterious side effects. There is therefore a desire to terminate treatment as soon as it is determined to have been successful. However, even when a patient has undetectable HCV RNA in serum, virological relapse may occur if therapy is discontinued. The detection of HCV RNA in serum is therefore not a reliable indicator that treatment may be stopped without relapse occurring.

The present inventors have found that the presence of HCV in white blood cells such as monocytes can be used to predict relapse in patients. HCV may be detected in white blood cells by, for example, detecting the presence or absence of HCV RNA.

In the method of the invention, a patient-derived white blood cell (such as a monocyte) is fused with a hepatocyte cell to produce a fused cell and the presence of HCV in the fused cell is investigated.

Measuring replicating HCV RNA in white blood cells may allow the duration of therapy to be optimised and reduced to the minimum needed to eradicate the virus.

### TREATMENT MONITORING

The method of the present invention may also be used to monitor the progression of an HCV treatment. In a patient undergoing HCV treatment, a white blood cell may be isolated from the patient at various time points during the course of the treatment and fused with a hepatocyte to make a fused cell.

Effectiveness of the treatment over time may be determined if the amount of HCV in the fused cell reduces over time.

Success of the treatment may be determined if, at a certain time point, no HCV is detectable in the fused cell.

The method of the invention may be used to determine when an HCV treatment may be terminated, at a time point at which HCV is undetectable in the fused cell produced from the white blood cell. Treatment may be continued until a plurality of (for example two or three) negative results are obtained, in order to increase the certainty that relapse will not occur.

The invention will now be further described by way of Examples, which are meant to serve to assist one of ordinary skill in the art in carrying out the invention and are not intended in any way to limit the scope of the invention.

### EXAMPLES

### Example 1 - Monocytes from patients with chronic HCV harbour replication competent HCV which is amplified following fusion

Patient derived monocytes from 12 patients with chronic HCV were extracted using commercial CD14 positive bead selection and fused to HuH7 cells (a hepatocyte cell line) using PEG fusion. The fused cells were cultured for varying periods and the amount of HCV RNA was detected using quantitative PCR. Whole cell mRNA was extracted using a standard phenol/chloroform extraction mixture and the amount of specific RNA was assessed using a standard qPCR detection assay with primers specific for HCV and Beta Actin. Amplified product was detected using the CyberGreen CT detection method and a Rotorgene PCR detection system as described by the machines manufacturers (Corbett Life Science). The quantity of HCV was expressed relative to Beta Actin. Controls were moncytes alone from the indicated patients and a genotype b replicon. As shown in Figure 1, replicating HCV was detected and an increase in HCV was observed compared to unfused monocytes. An increase in HCV RNA in fused cells was observed from about day 3 to day 7 compared to monocytes. From day 10 onwards the level of HCV RNA was similar to monocyte levels.

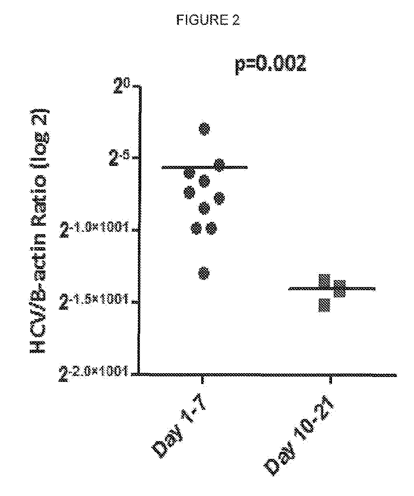

Figure 2 shows the results of a separate experiment in which patient derived monocytes from 13 patients with chronic HCV were fused to a hepatocyte cell line and the amount of HCV RNA was detected relative to Beta Actin mRNA at various time points. An increase in HCV RNA in fused cells was observed for up to 7 days, but the levels fell off after more than 7 days incubation.

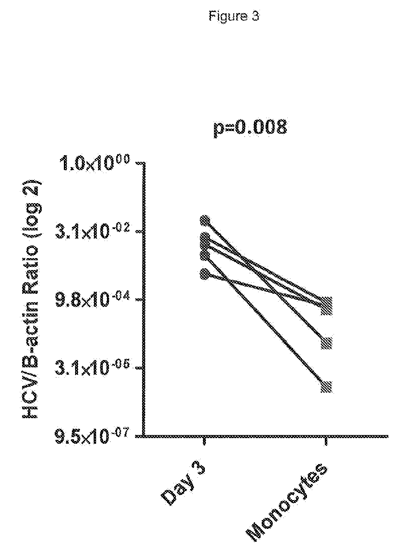

In order to show that HCV RNA is produced by fused monocytes, patient derived monocytes from 4 patients with chronic HCV were fused to an hepatocyte cell line and after 3 days the amount of HCV RNA was detected using quantitative PCR. Monocytes from the same patients were tested prior to fusion and the paired samples were linked graphically as shown in Figure 3.

### Example 2 - The fused cells can replicate HCV of any genotype.

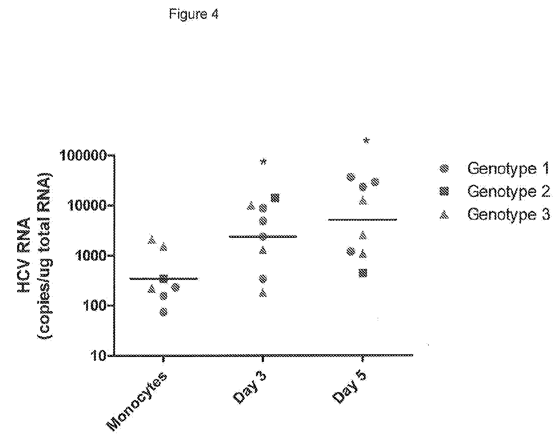

Eight patients with chronic HCV infection were selected: four of the patients were infected with HCV genotype 1; one patient was infected with HCV genotype; and three patients were infected with HCV genotype 3. Patient derived monocytes from each patient were fused to an hepatocyte cell line and the amount of HCV RNA was detected using quantitative PCR. The results are shown in Figure 4. This shows that fusion of patient-derived monocytes with hepatoma cells produces a fused cell capable of replicating HVC *in vitro* regardless of the HCV genotype.

### Example 3 - Fused monocytes show direct staining with antibodies against HCV

Five days after fusion the fused cells were stained with a commercial monoclonal antibody against human albumin and serum directed against the NS5A protein of HCV (a kind gift of Professor Mark Harris, University of Leeds). Cells were double stained with a fluorescence labelled secondary antibody and detected using immunofluorescence microscopy. For confocal microscopy fused cells along with negative and positive controls were viewed on Zeiss 510-META laser confocal microscope under an oil-immersion lens of x63 objective with numerical aperture being 1.40. Alexa-Fluor 488 (494 nm excitation; 519 nm emission) was excited using an argon laser fitted with 488 nm filter while Alexa fluor 565 was excited using argon laser which was fitted with 565 nm filter. Images are displayed as single optical sections of 40 µm thickness.
DAPI counter staining was applied to identify cells. The results are shown in Figure 5. Fused cells show direct staining with the HCV marker.

Confocal microscopy was also used to show co-staining of HCV NS5A and albumin in the fused cells (Figure 6).

Figure 9 also shows a HCV-patient-derived monocyte fused to a hepatocyte stains positively for HCV using an antibody directed against NS5A.

### Example 4 - The presence of replication competent HCV in monocytes at the end of therapy is associated with relapse

In patients with genotype 3 HCV who have cirrhosis the most common modality of treatment failure is relapse - i.e. the patient is HCV RNA undetectable in serum throughout therapy but viraemia returns when therapy is discontinued. The present inventors studied 16 patients with genotype 3 HCV and examined monocytes at the end of therapy in patients who did, or did not, relapse. Figure 7 shows that the presence of replication competent HCV RNA at the end of therapy was markedly increased in patients who relapse.

### Example 5 - Use of fused cells to show that the anti-viral effect of telaprevir is HCV genotype-specific

Patient derived monocytes from patients with genotype 1 and genotype 3 HCV were taken and incubated for a variety of different times (ranging from 3 to 7 days) with telaprevir at concentrations known to inhibit HCV RNA. The amount of HCV RNA was assessed using a standard qPCR technique and is expressed as the raw Ct value from the Corbett Rotorgene.

As shown in Figure 8, replication of HCV from patients with Genotype 1 HCV is inhibited by the antiviral agent telaprevir, whereas HCV Genotype 3 is not inhibited by telaprevir. This concurs with the clinical trial data showing the Genotype 1 HCV is sensitive to telaprevir whereas genotype 3 is not.

### Example 6 - A capture-fusion technique allows HCV in serum to be cultured

To determine whether circulating HCV could be 'captured' by monocytes, the present inventors cultured a commercially available monocyte cell line (ThP1) in the presence of a cocktail of pro-inflammatory cytokines. Pro-inflammatory cytokines were used as they may increase uptake of particles. Stimulated monocytes were cultured with serum from patients with HCV (diluted such that 10,000 HCV IU were added to 10,000 monocytes) and, after 24 hours were fused to hepatocytes, or left unfused. HCV replication was assessed after various time points. As shown in Figure 10, fusion of infected THP-1 cells enhances HCV replication.

Confocal microscopy of fused ThP1 cells with an antibody against human albumin and serum directed against the NS5A protein of HCV shows that these fused cells show direct staining with antibodies against HCV (Figure 11).

### Example 7 - Using fused cells to test the drug-sensitivity of a patient derived viral isolate

ThP1 cells in culture were cultured with serum from a patient with HCV and then fused to a hepatocyte cell line. The fused cells were treated for 5 days with the various putative anti-viral agents. HCV RNA was then assessed in the total cellular mRNA as described above. HCV RNA was quantified by using a cloned HCV cDNA diluted to a known concentration and used to generate a standard curve. The quantity of HCV RNA in the samples was calculated by comparing the Ct values to the Ct values from the standard curve. Results are expressed as the percentage of HCV RNA relative to untreated cells.

The results are shown in Figure 12 to 16, as shown in the following table:

| **Figure No** | **HCV genotype** | **Anti-viral drug** |
|---|---|---|
| 12 | 3 | Anti-viral compound A |
| 13 | 1 | Anti-viral compound A |
| 14 | 1 | IFN |
| 15 | 3 | IFN |
| 16 | 1 and 3 | Telaprevir |

Addition of drugs known to inhibit HCV replication showed that consistent dose response curves assessing the sensitivity of the cell to the drug in question could be obtained. This demonstrates that the fused cells of the present invention can be used to screen anti-HCV drugs *in vitro.*

### Example 8 - HCV quantitation

The procedure descibed in Example 6 was used to determine whether HCV RNA was quantifiable in serum from all patients and the results are shown in Figure 17. HCV RNA was quantifiable in serum from the majority of patients tested 25/28 patients (89%). The replication levels varied from patient to patient, however repeat experiments show that the level of replication for each patient was consistent.

### Example 9 - Investigating the effect of treatment with Telaprevir or Alisporivir on patients with different genotypes

The assay described in previous Examples was used to derive dose response curves from patients treated with different drugs. Sera from a patient with genotype 1 HCV and from a patient with genotype 3 HCV were used to infect THP-1 cells pre-stimulated with PMA (200ng/mL) and IFN-γ (10ng/mL). After 24 hours, the cells were thoroughly washed, fused with Huh-7.5 cells and then seeded into 6 well plates. Antiviral drugs (telaprevir or alisporivir) were added to the wells at a range of concentrations, as indicated in Figure 18, in quadruplicate. Drug dilution medium (RPMI/0.05% DMSO/2%FCS) was added to the "no drug" wells. Medium was changed and drug replenished after 3 days, and HCV RNA was quantified by qPCR after 5 days. One step reverse transcription-PCR was performed using the Qiagen Viral Nucleic Acid Detection Kit with a commercially available HCV primer-probe 6 set (Applied Biosystems). A standard curve ranging from 10 copies/reaction to 10 copies/reaction was included in each PCR run and used to calculate HCV copy number (Critical factors for successful real-time PCR. Qiagen, July 2010. Available at www.qiagen.com/literature/render.aspx?id=23490). To allow for differences in replication between viral isolates, HCV RNA was expressed as the percentage of that present in "no drug" wells. The results were used to construct dose-response curves for each isolate in response to each antiviral drug.

The assay of the present invention may therefore be used to monitor HCV levels in a patient following treatment and, importantly, to determine the point at which treatment may be safely stopped or needs to be substituted or complimented with an alternative treatment.

### Example 10 - Investigation of telaprevir/alisporivir sensitivity in a variety of different viral genotypes

Sera were obtained from patients infected with a variety of HCV genotypes (genotype 1, N = 9; genotype 2, N = 4; genotype 3, N = 5; genotype 4, N = 4; genotype 5, N = 2; genotype 6, N = 2). In separate experiments, these sera were used to infect THP-1 cells pre-stimulated with PMA (200ng/mL) and IFN-γ (10ng/mL) After 24 hours, the cells were thoroughly washed, fused with Huh-7.5 cells and then seeded into 6 well plates. Antiviral drugs (telaprevir or alisporivir) were added to the wells at a range of concentrations, in quadruplicate. Drug dilution medium (RPMI/0.05% DMSO/2%FCS) was added to the "no drug" wells. Medium was changed and drug replenished after 3 days, and HCV RNA was quantified by qPCR after 5 days. To allow for differences in replication between viral isolates, HCV RNA was expressed as the percentage of that present in "no drug" wells. Dose-response curves were constructed for each experiment using GraphPad Prism software and used to calculate the 50% inhibitory concentration (IC₅₀) of antiviral drug.

The assay of the invention can be used to assay telaprevir (Figure 19) or alisporivir (Figure 20) sensitivity in a variety of different HCV genotypes.

### Example 11 - Use of the assay to investigate reduced telaprevir sensitivity associated with NS3 mutations.

Using the methodology outlined in Example 10, the assay of the invention was used to assay telaprevir sensitivity in patients with telaprevir resistance associated NS3 mutations. The resluts are shown in Figure 21. Graph A shows mean±sem of capture-fusion experiments using pre-treatment and post-failure samples from 2 patients who failed therapy with telaprevir pegIFN and ribavirin. Both had wild-type NS3 pre-treatment and dual V36M/R155K telaprevir resistance mutations at treatment failure. Graph B shows telaprevir IC₅₀ for each patient before telaprevir treatment and after treatment failure.

These data demonstrate that known drug resistant mutations that reduce sensitivity to telaprevir can be detected with the assay of the invention.

As shown in Figure 22, the assay of the invention can also be used to identify of pre-existing telaprevir resistance even if, at the time of the assay the patient has wild-type NS3 (i.e. before acquisition of dual V36M/R155K telaprevir resistance mutations). The identification of pre-existing resistance enables pre-treatment identification of patients who are unlikely to respond to anti-viral therapy.

### Example 12 - Identification of pre-treatment telaprevir sensitivity in patients with G3 HCV

In a clinical trial 8 patients with genotype 3 HCV were treated with telaprevir. Three patients responded. Using conventional assays to identify telaprevir resistance (biochemical assays based on protease expression) it was impossible to identify sensitive and resistant patients (Foster et al (2011) Gastroenetrology 141:881-889).

Using the methodology described in previous Examples, it was investigated whether the capture fusion assay of the present invention could be used to identify pre-treatment telaprevir sensitivity in these patients. As shown in Figure 23, the assay of the invention_correctly identified the three patients who showed a clinical response to telaprevir. It is to be noted that the three patients who had a reduction in HCV RNA during treatment had a low IC50 when tested in the assay of the invention.

### Materials and Methods

### Cells

HuH 7.5 cells were maintained in Dulbecco's Modified Eagle Medium (DMEM) supplemented with 10% FCS and penicillin/streptomycin. THP-1 cells were maintained in RPMI supplemented with 10% fetal calf serum and penicillin/streptomycin.

Primary monocytes were obtained from patients on completion of 24 weeks of interferon and ribavirin therapy for treatment of genotype 3 HCV. 40mL whole blood was centrifuged on a Ficoll gradient. The PBMC layer was removed and washed twice in RPMI before the cells were stored in liquid nitrogen until use.

### Infection of THP-1 cells

THP-1 cells were seeded at a density of 1x10⁶ cells/mL and incubated with 10ng/mL interferon γ (IFNγ) and 200ng/mL PMA at 37°C for 24 hours. The supernatant was removed, cells washed with PBS and the medium replaced with RPMI supplemented with 2% FCS. Serum from an HCV positive donor was added at a ratio of one HCV IU per cell and incubated at 37°C overnight.

### Cell Fusion

*Primary monocyte.* CD14+ monocytes were positively selected from total PBMCs by magnetic cell separation, according to the manufacturer's instructions. The CD14+ cells obtained were counted and pelleted with an equal number of HuH 7.5 cells. The cell pellet was resuspended in pre-warmed polyethylene glycol 1500 (PEG 1500, Roche) over 1 minute then incubated at 37°C for 2 minutes. 10mL pre-warmed medium (DMEM supplemented with 10% FCS) was added over a further 3 minutes. The fused cells were incubated at 37°C for 5 minutes before a 5 minute centrifugation at 1000rpm. The cell pellet was resuspended in DMEM supplemented with 10% FCS and penicillin/streptomycin. Cells were seeded at a density of 5x10⁵ cells/mL then incubated at 37°C overnight, prior to addition of any antiviral compounds.

*THP-1 cells.* After overnight infection with serum containing HCV, the supernatant was removed and the THP-1 cells washed with PBS. The cells were removed from the wells using a cell scraper and pelleted together with an equal number of HuH 7.5 cells. The cells were then fused using PEG 1500, as described above. Fused cells were seeded at a density of 2x10⁵ cells/mL then incubated at 37°C overnight, prior to addition of any antiviral compounds.

### RNA extraction and RTqPCR

RNA was extracted from fused cells immediately after fusion and following a 5 day incubation using TRIzol reagent (Invitrogen). Total RNA obtained was quantified using the RiboGreen assay (Invitrogen). HCV RNA was measured by reverse transcription-quantitative PCR (RT-qPCR) using the Quantitect viral nucleic acid detection kit (Qiagen) in 20µL volumes with a TaqMan primer and probe for HCV detection (Applied Biosystems). 10-fold serial dilutions of PCR product corresponding to a 319 nucleotide segment between the 5'untranslated region and E1 region of the HCV genome were included as a quantitative standard. Final HCV copy number was expressed per microgram of total RNA.

### Immunofluorescence

Fused cells were seeded onto coverslips and grown for 3 or 5 days. The cells were fixed with 4% paraformaldehyde, permeabilised in 0.2% Triton X-100, blocked with 10% FCS and double stained with sheep anti-HCV NS5A and with rabbit anti-human albumin antibodies. Cells were then incubated with AlexaFluor488-conjugated donkey anti-sheep and AlexaFluor648-conjugated goat anti-rabbit antibodies, and examined under a Leica LS microscope.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology, virology or related fields are intended to be within the scope of the following claims.

## Claims

1. A method for replicating HCV virus *in vitro* which comprises the following steps:
(i) fusing an HCV-infected white blood cell with a hepatocyte cell to produce a fused cell; and
(ii) culturing the fused cell so that HCV replication may occur.

2. A fused cell capable of replicating HCV virus *in vitro* which is made by fusing an HCV-infected white blood cell with a hepatocyte cell.

3. A method for making a fused cell according to claim 2, which comprises the step of fusing an HCV-infected white blood cell with a hepatocyte cell *in vitro*.

4. A method according to claim 1 or 3, wherein the HCV-infected white blood cell is derived from a HCV patient.

5. A method according to claim 1 or 3, wherein the HCV-infected white blood cell is made by infecting a white blood cell with HCV virus *in vitro*.

6. A method according to claim 5, which comprises the following steps:
(i) culturing a white blood cell in vitro with serum from an HCV patient, so that the white blood cell is infected with HCV in the serum; and
(ii) fusing the HCV-infected white blood cell with a hepatocyte.

7. The use of a fused cell according to claim 2 to assess the capacity of a test HCV treatment to reduce HCV replication.

8. A method for screening a test compound or composition which comprises the step of analysing the capacity of the test compound or composition to reduce HCV replication in a fused cell according to claim 2.

9. A method according to claim 8, wherein the capacity of the test compound or composition is tested in a plurality of fused cells according to claim 2, each replicating a different strain of HCV virus, in order to analyse whether the effectiveness of the test compound or composition is HCV strain-specific.

10. An *in vitro* method for assessing the likelihood that a HCV patient will respond to a test compound or composition, which comprises the step of analysing the capacity of the test compound or composition to reduce HCV replication *in vitro* in a fused cell, wherein the fused cell is made by fusing a hepatocyte cell with a white blood cell which either:
(i) has been isolated from the HCV patient; or
(ii) is a white blood cell which has been infected with HCV virus from the subject *in vitro*.

11. An *in vitro* method for selecting a treatment which comprises the step of assessing the responsiveness of an HCV patient to a test compound or composition by a method according to claim 10 and selecting a test compound or composition which reduces HCV replication in the fused cell.

12. An *in vitro* method for assessing the likelihood that a subject will suffer from a HCV relapse following treatment,
which comprises the following steps:
(i) fusing a white blood cell which has been isolated from the subject with a hepatocyte cell to produce a fused cell; and
(ii) investigating whether HCV can be detected in the fused cell,
wherein a subject is determined to be likely to relapse if HCV is detectable in the fused cell.

13. An *in vitro* method for investigating the progression of an HCV treatment which comprises the following steps:
(i) fusing a white blood cell which has been periodically isolated from the subject with a hepatocyte cell to produce a fused cell; and
(ii) investigating whether HCV can be detected in the fused cell.

14. An *in vitro* method for investigating the progression of an HCV treatment which comprises the following steps:
(i) culturing a white blood cell in vitro with serum which has been periodically isolated from the subject, so that the white blood cell is infected with HCV in the serum; and
(ii) fusing the HCV-infected white blood cell with a hepatocyte; and
(iii) quantifying HCV RNA in the fused cell.

15. An *in vitro* method for determining when an HCV treatment may be stopped by investigating the progression of the treatment by a method according to claim 13 or 14 and determining that the treatment may be stopped when a white blood cell or a serum sample is isolated which produces a fused cell in which HCV is undetectable.

## Patentansprüche

1. Verfahren zum Replizieren von HCV-Viren *in vitro,* welches die folgenden Schritte umfasst:
(i) Fusionieren HCV-infizierter weißer Blutkörperchen mit einer Hepatozytenzelle, um eine fusionierte Zelle zu erhalten; und
(ii) Anzüchten der fusionierten Zelle, derart, dass es zu einer HCV-Replizierung kommen kann.

2. Fusionierte Zelle, die dazu befähigt ist, das HCV-Virus *in vitro* zu replizieren, wobei sie hergestellt wird, indem ein HCV-infiziertes weißes Blutkörperchen mit einer Hepatozytenzelle fusioniert wird.

3. Verfahren zur Herstellung einer fusionierten Zelle nach Anspruch 2, welches den Schritt des Fusionierens eines HCV-infizierten weißen Blutkörperchens *in vitro* mit einer Hepatozytenzelle umfasst.

4. Verfahren nach Anspruch 1 oder 3, wobei das HCVinfizierte weiße Blutkörperchen von einem HCV-Patienten stammt.

5. Verfahren nach Anspruch 1 oder 3, wobei das HCVinfizierte weiße Blutkörperchen hergestellt wird, indem ein weißes Blutkörperchen *in vitro* mit einem HCV-Virus infiziert wird.

6. Verfahren nach Anspruch 5, welches die folgenden Schritte umfasst:
(i) Anzüchten eines weißen Blutkörperchens in vitro mit Serum von einem HCV-Patienten, derart, dass das weiße Blutkörperchen mit dem HCV infiziert wird, der in dem Serum vorliegt; und
(ii) Fusionieren des HCV-infizierten weißen Blutkörperchens mit einem Hepatozyten.

7. Verwenden einer fusionierten Zelle nach Anspruch 2, um die Fähigkeit einer zu prüfenden HCV-Behandlung zur Verringerung der HCV-Replizierung zu bewerten.

8. Verfahren zum Screening einer zu prüfenden Verbindung oder Zusammensetzung, welches den Schritt des Analysierens der Fähigkeit der zu prüfenden Verbindung oder Zusammensetzung zur Verringerung der HCV-Replizierung in einer fusionierten Zelle nach Anspruch 2 umfasst.

9. Verfahren nach Anspruch 8, wobei die Fähigkeit der zu prüfenden Verbindung oder Zusammensetzung in mehreren fusionierten Zellen nach Anspruch 2 geprüft wird, wobei jede davon einen unterschiedlichen Stamm des HCV-Virus repliziert, um zu analysieren, ob die Wirksamkeit der zu prüfenden Verbindung oder Zusammensetzung HCV-Stammspezifisch ist.

10. In-vitro-Verfahren zur Bewertung der Wahrscheinlichkeit, dass ein HCV-Patient auf eine zu prüfende Verbindung oder Zusammensetzung ansprechen wird, welches den Schritt des Analysierens der Fähigkeit der zu prüfenden Verbindung oder Zusammensetzung zur Verringerung der HCV-Replizierung *in vitro* in einer fusionierten Zelle umfasst, wobei die fusionierte Zelle hergestellt wird, indem eine Hepatozytenzelle mit einem weißen Blutkörperchen verschmolzen wird, welches entweder:
(i) aus einem HCV-Patienten isoliert wurde; oder
(ii) bei dem es sich um ein weißes Blutkörperchen handelt, welches *in vitro* mit einem HCV-Virus aus diesem Patienten infiziert wurde.

11. *In vitro* Verfahren zur Auswahl einer Behandlung, welches den Schritt des Bewertens des Ansprechverhaltens eines HCV-Patienten auf eine zu prüfende Verbindung oder Zusammensetzung mittels eines Verfahrens nach Anspruch 10 und des Auswählens einer zu prüfenden Verbindung oder Zusammensetzung, welche die HCV-Replizierung in der fusionierten Zelle verringert, umfasst.

12. In-vitro-Verfahren zur Bewertung der Wahrscheinlichkeit, dass ein Patient nach einer Behandlung einen HCV-Rückfall erleiden wird,
wobei es die folgenden Schritte umfasst:
(i) Fusionieren eines weißen Blutkörperchens, das aus dem Patienten isoliert wurde, mit einer Hepatozytenzelle, um eine fusionierte Zelle zu erhalten; und
(ii) Untersuchen, ob HCV in der fusionierten Zelle nachgewiesen werden kann,
wobei es für wahrscheinlich befunden wird, dass der Patient einen Rückfall erleidet, wenn HCV in der fusionierten Zelle nachgewiesen werden kann.

13. *In-vitro*-Verfahren zur Untersuchung des Voranschreitens einer HCV-Behandlung, welches die folgenden Schritte umfasst:
(i) Fusionieren eines weißen Blutkörperchens, das in regelmäßigen Abständen aus dem Patienten isoliert wurde, mit einer Hepatozytenzelle, um eine fusionierte Zelle herzustellen; und
(ii) Untersuchen, ob HCV in der fusionierten Zelle nachgewiesen werden kann.

14. *In-vitro*-Verfahren zur Untersuchung des Voranschreitens einer HCV-Behandlung, welches die folgenden Schritte umfasst:
(i) Anzüchten eines weißen Blutkörperchens in vitro mit Serum, das in regelmäßigen Abständen aus einem Patienten isoliert wurde, derart, dass das weiße Blutkörperchen mit dem HCV infiziert wird, der in dem Serum vorliegt; und
(ii) Fusionieren des HCV-infizierten weißen Blutkörperchens mit einem Hepatozyten; und
(iii) Quantifizieren der HCV-RNA in der fusionierten Zelle.

15. *In-vitro*-Verfahren zur Bestimmung des Zeitpunkts, an welchem eine HCV-Behandlung abgebrochen werden kann, wobei zu diesem Zwecke das Voranschreiten der Behandlung mittels eines Verfahrens nach Anspruch 13 oder 14 untersucht wird und befunden wird, dass die Behandlung abgebrochen werden kann, wenn ein weißes Blutkörperchen oder eine Serumprobe isoliert wird, das/die eine fusionierte Zelle hervorbringt, in welcher kein HCV nachgewiesen werden kann.

## Revendications

1. Procédé pour la réplication du virus VHC *in vitro* qui comprend les étapes suivantes :
(i) la fusion d'un globule blanc infecté par le VHC avec une cellule hépatocyte pour produire une cellule fusionnée ; et
(ii) la culture de la cellule fusionnée de sorte que la réplication du VHC peut avoir lieu.

2. Cellule fusionnée capable de répliquer le virus VHC *in vitro* qui est formée par fusion d'un globule blanc infecté par le VHC avec une cellule hépatocyte.

3. Procédé pour la formation d'une cellule fusionnée selon la revendication 2, qui comprend l'étape de fusion d'un globule blanc infecté par le VHC avec une cellule hépatocyte *in vitro.*

4. Procédé selon la revendication 1 ou 3, dans lequel le globule blanc infecté par le VHC est issu d'un patient infecté par le VHC.

5. Procédé selon la revendication 1 ou 3, dans lequel le globule blanc infecté par le VHC est formé par infection d'un globule blanc avec le virus VHC *in vitro.*

6. Procédé selon la revendication 5, qui comprend les étapes suivantes :
(i) la culture d'un globule blanc *in vitro* avec du sérum provenant d'un patient infecté par le VHC, de sorte que le globule blanc est infecté par le VHC dans le sérum ; et
(ii) la fusion du globule blanc infecté par le VHC avec un hépatocyte.

7. Utilisation d'une cellule fusionnée selon la revendication 2 pour évaluer la capacité d'un traitement contre le VHC d'essai à réduire la réplication du VHC.

8. Procédé pour le criblage d'un composé ou d'une composition d'essai qui comprend l'étape d'analyse de la capacité du composé ou de la composition d'essai à réduire la réplication du VHC dans une cellule fusionnée selon la revendication 2.

9. Procédé selon la revendication 8, dans lequel la capacité du composé ou de la composition d'essai est testée dans une pluralité de cellules fusionnées selon la revendication 2, chacune répliquant une souche différente du virus VHC, afin d'analyser si l'efficacité du composé ou de la composition d'essai est spécifique à une souche du VHC.

10. Procédé *in vitro* pour l'évaluation de la probabilité qu'un patient infecté par le VHC réponde à un composé ou une composition d'essai, qui comprend l'étape d'analyse de la capacité du composé ou de la composition d'essai à réduire la réplication du VHC *in vitro* dans une cellule fusionnée, dans lequel la cellule fusionnée est formée par fusion d'une cellule hépatocyte avec un globule blanc qui soit :
(i) a été isolé à partir du patient infecté par le VHC ; soit
(ii) est un globule blanc qui a été infecté par le virus VHC provenant du sujet *in vitro.*

11. Procédé *in vitro* pour la sélection d'un traitement qui comprend l'étape d'évaluation de la sensibilité d'un patient infecté par le VHC à un composé ou une composition d'essai par un procédé selon la revendication 10 et de sélection d'un composé ou d'une composition d'essai qui réduit la réplication du VHC dans la cellule fusionnée.

12. Procédé *in vitro* pour l'évaluation de la probabilité qu'un sujet souffre d'une rechute d'infection par le VHC après traitement, qui comprend les étapes suivantes :
(i) la fusion d'un globule blanc qui a été isolé à partir du sujet avec une cellule hépatocyte pour produire une cellule fusionnée ; et
(ii) le fait d'étudier si le VHC peut être détecté dans la cellule fusionnée,
dans lequel un sujet est déterminé être susceptible de rechute si le VHC est détectable dans la cellule fusionnée.

13. Procédé *in vitro* pour l'étude de la progression d'un traitement contre le VHC qui comprend les étapes suivantes :
(i) la fusion d'un globule blanc qui a été périodiquement isolé à partir du sujet avec une cellule hépatocyte pour produire une cellule fusionnée ; et
(ii) le fait d'étudier si le VHC peut être détecté dans la cellule fusionnée.

14. Procédé *in vitro* pour l'étude de la progression d'un traitement contre le VHC qui comprend les étapes suivantes :
(i) la culture d'un globule blanc *in vitro* avec du sérum qui a été périodiquement isolé à partir du sujet, de sorte que le globule blanc est infecté par le VHC dans le sérum ; et
(ii) la fusion du globule blanc infecté par le VHC avec un hépatocyte ; et
(iii) la quantification d'ARN de VHC dans la cellule fusionnée.

15. Procédé *in vitro* pour la détermination du moment où un traitement contre le VHC peut être arrêté par l'étude de la progression du traitement par un procédé selon la revendication 13 ou 14 et la détermination que le traitement peut être arrêté lorsqu'on isole un globule blanc ou un échantillon de sérum qui produit une cellule fusionnée dans laquelle le VHC est indétectable.
